# EUROPEAN PATENT SPECIFICATION

(11) **EP 1 864 133 B1**
(45) Date of publication and mention of the grant of the patent: **10.03.2010**
(21) Application number: 06739883.4
(22) Date of filing: 30.03.2006
(51) Int. Cl.: G01N 33/569, G01N 33/53

(54) **METHOD FOR BREASTCANCER PROGNOSIS USING CELLULAR FOLATE VITAMIN RECEPTOR QUANTIFICATION**
VERFAHREN ZUR PROGNOSE VON BRUSTKREBS MITTELS QUANTIFIZIERUNG VON ZELLULÄREN FOLAT VITAMIN REZEPTOREN
METHODE DESTINEE A PRONOSTIQUER UN CANCER DU SEIN PAR QUANTIFICATION DES RECEPTEURS DES FOLATES CELLULAIRES

(30) Priority: 30.03.2005 US 666430 P
(43) Date of publication of application: 12.12.2007
(73) Proprietor: Purdue Research Foundation, West Lafayette, IN 47906 (US); Endocyte, Inc., West Lafayette, IN 47906 (US); MAYO FOUNDATION FOR MEDICAL EDUCATION AND RESEARCH, Rochester, Minnesota 55905 (US)
(72) Inventor: LOW, Philip, Stewart, West Lafayette, Indiana 47906 (US); HARTMANN, Lynn, C., Rochester, Minnesota 55902 (US); LEAMON, Christopher, Paul, West Lafayette, Indiana 47906 (US); ELLIS, Phil, Ronald, West Lafayette, Indiana 47906 (US)
(74) Representative: Elsy, David
(86) International application number: PCT/US2006/011376
(87) International publication number: WO 2006/105141

(56) References cited:
- TOFFOLI GIUSEPPE ET AL: "Expression of folate binding protein as a prognostic factor for response to platinum-containing chemotherapy and survival in human ovarian cancer" INTERNATIONAL JOURNAL OF CANCER, vol. 79, no. 2, 17 April 1998 (1998-04-17), pages 121-126, XP002394383 ISSN: 0020-7136
- PARKER N ET AL: "Folate receptor expression in carcinomas and normal tissues determined by a quantitative radioligand binding assay" ANALYTICAL BIOCHEMISTRY, ACADEMIC PRESS, NEW YORK, NY, US, vol. 338, no. 2, 15 March 2005 (2005-03-15), pages 284-293, XP004770829 ISSN: 0003-2697
- CERNIGOI C ET AL: "Folate binding protein as a marker for survival in ovarian cancer patients and a predictor of response to chemotherapy" CELL PROLIFERATION 1997 UNITED KINGDOM, vol. 30, no. 10-12, 1997, page 468, XP009070977 ISSN: 0960-7722
- TOFFOLI GIUSEPPE ET AL: "Prognostic significance of folate binding protein in ovarian neoplasms" TUMORI, vol. 85, no. 3 SUPPL. 1, May 1999 (1999-05), pages S39-S41, XP009070976 ISSN: 0300-8916
- HOLM JAN ET AL: "Folate receptor of human mammary adenocarcinoma" APMIS, vol. 102, no. 6, 1994, pages 413-419, XP009070950 ISSN: 0903-4641

## Description

### FIELD OF THE DISCLOSURE

This invention relates to methods and kits for obtaining a prognosis for a cancer by quantification of vitamin receptor expression levels in the cancer cells and to guide the management or develop an effective treatment for the cancer. The invention also relates to methods and kits for determining the presence of vitamin receptors on cancer cells to select patients that should be treated with a therapy that utilizes vitamin receptor targeting.

### BACKGROUND AND SUMMARY

Effective treatment regimens for cancers often depend on the ability to obtain a reliable prognosis for the cancer so that the most effective treatment regimen for the patient can be developed. An important clinical priority is to improve prognostic capabilities for cancers, and to develop molecular-based therapeutic approaches to improve patient management and treatment. Determination of which prognostic group a patient diagnosed with cancer falls into is critical in determining an optimal treatment regimen and, thus, is critical to patient survival.

For example, breast cancer is the most commonly diagnosed life threatening malignancy in North America. Breast cancer is the leading cause of death for women between 30-50 years of age in the United States and more than 200,000 new cases occur in the United States each year. Tumor size and nodal status are still the most reliable methods for predicting outcome although tumor grade, nuclear grade, histologic type, DNA ploidy, and hormone receptor status are also used. Only a few tumor markers have been identified for breast cancer, and most of those markers are not reliable enough to be used in prognostic assays. Accordingly, there is a critical need for better prognostic assays for breast cancer, and for other cancers.

Furthermore, selecting patients that should be treated with a particular therapy can depend on the ability to detect the presence of a tumor marker on the surface of a tumor so it can be determined whether a therapy that targets that tumor marker is warranted. For example, human epidermal growth factor receptor 2 (HER2) overexpression occurs in about 25% of breast cancer patients. Herceptin® (Genentech, Inc., San Francisco, CA), a monoclonal antibody directed to the HER2 protein, has been developed as a breast cancer therapy. Herceptin® is not administered to breast cancer patients, however, until the HER2 status of the breast cancer patient is determined. If the breast cancer patient is HER2 positive, Herceptin® treatment is warranted.

Vitamin receptors are overexpressed on cancer cells. For example, the high affinity folate receptor is a membrane-associated glycoprotein identified as a monoclonal antibody-defined antigen in placenta and trophoblastic cells. The high affinity folate receptor is rarely expressed or is nondetectable in most normal cells. However, it is overexpressed or preferentially expressed in cancers of epithelial origin, perhaps providing a growth advantage to these malignant cells. A high level of expression of the folate receptor is detectable in > 90% of ovarian cancers, and lesser degrees of positivity have been detected in endometrial, breast, renal, lung, brain, uterine, pancreatic, bladder, testicular, and colorectal cancers, and lymphomas, and other head and neck cancers.

For example, varying results have been obtained from studies of folate receptor expression in breast cancers, depending on the techniques used and the tissues analyzed. Ross et al. measured mRNA for the folate receptor and found levels to be elevated in five cancers when compared with normal breast specimens. However, the degree of elevation was approximately ten-fold less than that seen in ovarian cancer (Ross et al., Cancer 73: 2432-2443 (1994)). Garin-Chesa et al. used the mouse monoclonal antibody LK26 to assess folate receptor expression in a variety of fresh-frozen cancers. Of fifty-three breast cancers studied, two showed homogeneous LK26 staining and another nine cancers showed patchy staining (Garin-Chesa et al., Am. J. Pathol. 142(2): 557-67 (1993)).

Applicants have investigated the use of vitamin receptor overexpression in cancer cells in an assay for obtaining a prognosis for cancers and to guide the management or develop an effective treatment regimen for the patient. This method employs the quantification of vitamin receptor overexpression in cancer cells. Applicants have also investigated the use of methods to determine the presence of vitamin receptors in cancer cells for selecting patients that should be treated with a therapy that utilizes vitamin receptor targeting.

In one illustrative embodiment of the invention, a method is provided for determining a prognosis for a cancer by quantifying vitamin receptor expression in the cancer cells. The method comprises the steps of quantifying vitamin receptor expression in the cancer cells, and determining a prognosis for the cancer. In another illustrative embodiment, the vitamin receptor can be a folate receptor. In yet another embodiment, the cancer cells can be breast cancer cells or the cancer cells can be selected from the group consisting of ovarian cancer cells, uterine cancer cells, endometrial cancer cells, colorectal cancer cells, brain cancer cells, renal cancer cells, lymphoma cells, and lung cancer cells. In the embodiment where the cancer cells are breast cancer cells, the breast cancer can comprise node-negative disease, but the invention is not limited to node-negative disease. The method can further comprise the step of determining a treatment regimen for the cancer.

In another illustrative embodiment, the quantifying step can comprise immunohistochemical staining using an antibody. In this embodiment, the antibody can be a polyclonal antibody, or a mixture thereof, or a monoclonal antibody, or a mixture thereof, or polyclonal and monoclonal antibodies in combination. Alternatively, or additionally, the quantifying step can comprise in situ hybridization or receptor quantification using a radioreceptor assay that employs a radiolabeled ligand.

In another illustrative embodiment of the invention, an immunohistochemical method for determining a prognosis for a cancer is provided. The method comprises the steps of contacting the cancer cells with an antibody directed to a vitamin receptor, quantifying vitamin receptor expression on the cancer cells, and determining a prognosis for the cancer. In one illustrative embodiment, the vitamin receptor can be a folate receptor. In another embodiment, the cancer cells can be breast cancer cells. In this embodiment, the breast cancer can be node-negative disease, but the invention is not limited to node-negative disease. In yet another embodiment, the cancer cells can be selected from the group consisting of ovarian cancer cells, uterine cancer cells, endometrial cancer cells, colorectal cancer cells, brain cancer cells, renal cancer cells, melanoma cells, multiple myeloma cells, lymphoma cells, and lung cancer cells.

In other illustrative embodiments, the antibody can be a polyclonal antibody, or a mixture thereof, or a monoclonal antibody, or a mixture thereof, or polyclonal and monoclonal antibodies in combination.

In another embodiment, a method is provided for determining a treatment regimen for a cancer patient selected for treatment with a therapy that utilizes vitamin receptor targeting. The method comprises the steps of contacting cancer cells from the patient with an antibody directed to the vitamin receptor, quantifying vitamin receptor expression on the cancer cells, and determining a treatment regimen for the cancer patient.

In another illustrative embodiment of the invention, an in situ hybridization method for determining a prognosis for a cancer is provided. The method comprises the steps of contacting the cancer cells with a nucleic acid probe wherein the nucleic acid probe hybridizes to a nucleic acid that encodes the vitamin receptor or hybridizes to a nucleic acid that is complementary to the nucleic acid that encodes the vitamin receptor, quantifying vitamin receptor expression in the cancer cells, and determining a prognosis for the cancer.

In one illustrative embodiment, the vitamin receptor can be a folate receptor. In another illustrative embodiment, the cancer cells can be breast cancer cells. In this embodiment, the breast cancer can comprise node-negative disease, but the invention is not limited to node-negative disease. In yet another illustrative embodiment, the cancer cells can be selected from the group consisting of ovarian cancer cells, uterine cancer cells, endometrial cancer cells, colorectal cancer cells, brain cancer cells, renal cancer cells, melanoma cells, multiple myeloma cells, lymphoma cells, and lung cancer cells.

In another illustrative embodiment of the invention, a method is provided for determining a prognosis for a cancer. The method comprises the steps of contacting the cancer cells with a radiolabeled vitamin receptor-binding ligand, or an analog thereof, quantifying the number of vitamin receptors on the cancer cells, and determining a prognosis for the cancer.

In one illustrative embodiment, the vitamin receptor can be a folate receptor. In another illustrative embodiment, the cancer cells can be breast cancer cells. In this embodiment, the breast cancer can comprise node-negative disease, but the invention is not limited to node-negative disease. In yet another illustrative embodiment, the cancer cells can be selected from the group consisting of ovarian cancer cells, uterine cancer cells, endometrial cancer cells, colorectal cancer cells, brain cancer cells, renal cancer cells, melanoma cells, multiple myeloma cells, lymphoma cells, and lung cancer cells. In another illustrative embodiment the radiolabeled ligand can be radiolabeled folate, or an analog thereof.

In another illustrative embodiment of the invention, a kit is provided for use in performing an immunohistochemical staining assay. The kit comprises calibration micrographs wherein the calibration micrographs are derived from cancer tissues stained with an antibody to a vitamin receptor. In one illustrative embodiment, the kit can further comprise an antibody to a vitamin receptor. In another embodiment the kit can further comprise reagents for immunohistochemical staining. In another embodiment, the kit can comprise instructions for use of the calibration micrographs and for performing the immunohistochemical staining assay.

In still another embodiment of the invention, a kit is provided for performing a fluorescence in situ hybridization assay. In this illustrative embodiment, the kit comprises calibration micrographs where the calibration micrographs are derived from cancer tissues where nucleic acids from the cancer tissues have been hybridized in situ with a fluorescently-labeled nucleic acid probe. The nucleic acid probe hybridizes to a nucleic acid that encodes a vitamin receptor or the probe hybridizes to a nucleic acid that is complementary to the nucleic acid that encodes the vitamin receptor. In another embodiment, the kit can further comprise the fluorescently-labeled nucleic acid probe. In another embodiment, the kit can comprise reagents for in situ hybridization. In yet another embodiment, the kit can comprise instructions for use of the calibration micrographs and for performing the fluorescence in situ hybridization assay.

In another illustrative embodiment, a kit is provided for performing a vitamin receptor-binding assay. In this illustrative embodiment, the kit comprises a calibration table where the calibration table specifies ranges of numbers of vitamin receptors on the cancer cells wherein the ranges are correlated with a good versus a poor outcome for the cancer. In another illustrative embodiment, the kit can further comprise a radiolabeled vitamin receptor-binding ligand, or an analog thereof. In another illustrative embodiment, the kit can further comprise reagents for performing the vitamin receptor-binding assay. In another illustrative embodiment, the kit can further comprise instructions for performing the vitamin receptor-binding assay.

### BRIEF DESCRIPTION OF THE DRAWINGS

Fig. 1 shows an example of intensity 1+ immunohistochemical staining of folate receptors in breast cancer cells, which comprises weak, finely granular staining.
Fig. 2 shows an example of intensity 2+ immunohistochemical staining of folate receptors in breast cancer cells, which comprises coarse, granular staining.
Fig. 3 shows an example of intensity 3+ immunohistochemical staining of folate receptors in breast cancer cells, which comprises strong, intense, coarsely granular staining.
Fig. 4 shows an example of disease recurrence versus average intensity of folate receptor staining.
Fig. 5 shows examples of immunohistochemical staining of an adenocarcinoma of the pancreas with a monoclonal antibody (mAb 343; panels A and B) to folate receptor alpha. Panels A and B show membranous staining of the apical surface of columnar cells (open arrows) and panel B shows granular cytoplasmic staining of the columnar cells (filled arrow). Panel C shows a section incubated with non-immune mIgG₁.
Fig. 6 shows examples of immunohistochemical staining of an endometriod carcinoma with a monoclonal antibody (mAb 343; panels A and B) to folate receptor alpha. Panels A and B show intense cytoplasmic and apical membranous staining (open arrows) in glandular epithelial cells. No staining is shown in the stroma between the glands (filled arrow in panel B). Panel C shows a section incubated with non-immune mIgG₁.
Fig. 7 shows examples of immunohistochemical staining of a squamous cell carcinoma of the cervix with a monoclonal antibody (mAb 343; panels A and B) to folate receptor alpha. Focal cytoplasmic staining of squamous cells was seen (open arrow in panel B). Panel C shows a section incubated with non-immune mIgG₁.

### DETAILED DESCRIPTION

Methods and kits are provided for obtaining a prognosis for a cancer by quantification of vitamin receptor expression in the cancer cells and to guide the management or develop an effective treatment for the cancer. Methods and kits are also provided for determining the presence of vitamin receptors (i.e., detecting vitamin receptors) in cancer cells to select patients that should be treated with a therapy that utilizes vitamin receptor targeting. In illustrative embodiments, the vitamin receptor can be a folate receptor, the cancer cells can be selected from the group consisting of ovarian cancer cells, uterine cancer cells, endometrial cancer cells, colorectal cancer cells, brain cancer cells, renal cancer cells, melanoma cells, multiple myeloma cells, lymphoma cells, and lung cancer cells. The cancer tissues for use in the methods can be surgically removed from the patient. In the embodiment where the cancer cells are breast cancer cells, the breast cancer can comprise node-negative disease, but the invention is not limited to node-negative disease. The method can be performed on the primary malignant mass and can have prognostic value for metastatic disease.

In other illustrative embodiments, vitamin receptor expression can be quantified or detected by using such techniques as an immunohistochemical staining method, a fluorescent in situ hybridization method, Southern blotting, dot blot hybridizations, radioreceptor assays using a radiolabeled ligand, and the like. In the embodiment where an immunohistochemical staining method is used, the antibody used can be a polyclonal antibody, or a mixture thereof, or a monoclonal antibody, or a mixture thereof, or polyclonal and monoclonal antibodies in combination.

In still other illustrative embodiments an immunohistochemical staining method or an in situ hybridization method or a radioreceptor assay using a radiolabeled ligand is provided that uses any of the above-described features.

In another illustrative embodiment of the invention, a kit is provided for performing an immunohistochemical staining assay. The kit comprises calibration micrographs wherein the calibration micrographs are derived from cancer tissues stained with an antibody to a vitamin receptor. In one illustrative embodiment, the kit can further comprise an antibody to a vitamin receptor. In another embodiment the kit can further comprise reagents for immunohistochemical staining. In another embodiment, the kit can comprise instructions for use of the calibration micrographs and/or for performing the immunohistochemical staining assay.

In still another embodiment of the invention, a kit is provided for performing fluorescence in situ hybridization. In this illustrative embodiment, the kit comprises calibration micrographs where the calibration micrographs are derived from cancer tissues where nucleic acids from the cancer tissues have been hybridized in situ with a fluorescently-labeled nucleic acid probe. The nucleic acid probe hybridizes to a nucleic acid that encodes a vitamin receptor or the probe hybridizes to a nucleic acid that is complementary to the nucleic acid that encodes the vitamin receptor. In another embodiment, the kit can further comprise the fluorescently-labeled nucleic acid probe. In another embodiment, the kit can comprise reagents for in situ hybridization. In yet another embodiment, the kit can comprise instructions for use of the calibration micrographs and/or for performing the fluorescence in situ hybridization assay.

In another illustrative embodiment, a kit is provided for performing a vitamin receptor-binding assay. In this illustrative embodiment, the kit comprises a calibration table where the calibration table specifies ranges of numbers of vitamin receptors on the cancer cells wherein the ranges are correlated with a good versus a poor outcome for the cancer. In another illustrative embodiment, the kit can further comprise a radiolabeled vitamin receptor-binding ligand, or an analog thereof. In another illustrative embodiment, the kit can further comprise reagents for performing the vitamin receptor-binding assay. In another illustrative embodiment, the kit can further comprise instructions for use of the calibration table and/or for performing the vitamin receptor-binding assay.

In accordance with the invention, "quantifying" or "quantify" as used herein means determining the number of vitamin receptors on the cancer cells or means determining the level of vitamin receptor expression in the cancer cells, directly or indirectly. Examples of the meaning of "quantifying" or "quantify" as used herein can be found in Examples 5, 8, 9, and 10 where immunohistochemical staining (Examples 5, 8, and 9) is assigned a staining intensity of 1+, 2+, or 3+. For fluorescence in situ hybridization (FISH), for example, amplification of the vitamin receptor gene is quantified by counting signals in nuclei representing the presence of a vitamin receptor gene and comparing the number of vitamin receptor gene signals to the number of signals for a gene that is not amplified to obtain a ratio of amplified to nonamplified gene signals (Example 10). Such methods are known in the art for the quantification of amplification of the HER-2/neu gene. "Quantifying" or "quantify" can also mean determining a more absolute number of vitamin receptors on the cancer cells by, for example, using a vitamin receptor-binding assay that employs a radiolabeled ligand (Example 11).

There is a need for the type of "quantifying" described herein so that the physician can place the patient in a group such as a "good outcome" or a "poor outcome" group, depending on the immunohistochemical staining intensity or fluorescence intensity or the receptor number determined using a vitamin receptor-binding assay, to allow the physician to determine the most effective treatment regimen for the patient. A high (for example 2+ or 3+ immunohistochemical staining intensity or fluorescence intensity) correlates with a poor outcome and such patients should receive an aggressive treatment regimen.

The method and kits of the present invention can be used for both human clinical medicine and veterinary medicine applications. The methods and kits described herein may be used alone, or in combination with other prognostic methods or prognostic indicators (such as those described herein) or prognostic kits or kits for developing an effective therapy for a cancer or with methods or kits for determining the presence of vitamin receptors on cancer cells to select patients that should be treated with a therapy that utilizes vitamin receptor targeting. When vitamin receptor expression alone is used to determine a prognosis, without another prognostic indicator, vitamin receptor expression is considered an independent prognostic factor.

The cancer cells can be a cancer cell population that is tumorigenic, including benign tumors and malignant tumors (e.g., metastatic), or the cancer cells can be non-tumorigenic. The cancer cell population may arise spontaneously or by such processes as mutations present in the germline of the host animal or somatic mutations, or the cancer cell population may be chemically-, virally-, or radiation-induced. The invention can be used to obtain a prognosis for or to detect vitamin receptors on such cancers as carcinomas, sarcomas, lymphomas, Hodgekin' s disease, melanomas, mesotheliomas, Burkitt's lymphoma, nasopharyngeal carcinomas, leukemias, and myelomas (e.g., multiple myeloma). The cancer cell population can include, but is not limited to, brain, oral, thyroid, endocrine, skin, gastric, esophageal, endometrial, laryngeal, other head and neck, pancreatic, colon, colorectal, bladder, bone, ovarian (e.g., serous, endometrioid, and mucinous), cervical, uterine, breast, testicular, prostate, rectal, kidney, liver, and lung cancers (e.g., adenocarcinoma and mesothelioma), or any other cancer that overexpresses vitamin receptors.

Most of the types of cancers in the preceding paragraph are, for example, known to be vitamin receptor positive (see Ross et al., Cancer 73: 2432-2443 (1994); Garin-Chesa et al., Am. J. Pathol. 142(2): 557-67 (1993); Franklin, et al., Int. J. Cancer Suppl. 8: 89-95 (1994); Li et al., J. Nuc. Med. 37:665-672 (1996); Toffoli, Int. J. Cancer 74: 193-198 (1997); Veggian, Tumor 75:510-513 (1989); Weitman, et al., Cancer Research 52: 3396-3401; and Bueno et al., J. Thor. Card. Sur. Feb. 121: 225-233 (2001)).

The therapeutic regimen that is developed as a result of obtaining a cancer prognosis can include, for example, the vitamin receptor targeting therapies described in U.S. Patent Application Publications Nos. US-2001-0031252-A1; US-2003-0086900-A1; US-2003-0198643-A1; US-2005-0002942-A1; or PCT International Publication No. WO 03/097647. or a combination of these therapies. The therapeutic regimen that is developed can also include radiation therapy, chemotherapy, immunotherapy, or aggressive monitoring, or a combination of these therapies, if the patient falls into the poor outcome group.

Alternatively, a less aggressive approach can be used for patients that fall into the good outcome group. More aggressive therapies are required when, for example, strong staining or fluorescence (e.g., 2+ or 3+) is observed using the methods described herein or when a threshold number of receptors or amplified genes is quantified in or on the cancer cells using a FISH assay or a vitamin receptor-binding assay, respectively.

In one illustrative embodiment, the cancer cells can be selected from the group consisting of ovarian cancer cells, uterine cancer cells, endometrial cancer cells, colorectal cancer cells, brain cancer cells, renal cancer cells, melanoma cells, multiple myeloma cells, lymphoma, and lung cancer cells, or any other cancer that overexpresses vitamin receptors. In the embodiment where the cancer cells are breast cancer cells, the breast cancer can comprise node-negative disease, but the invention is not limited to node-negative disease.

The antibodies for use in the methods and kits for the immunohistochemical staining assay described herein can be polyclonal or monoclonal antibodies (e.g., PU17 or mAb 343). In illustrative embodiments, a mixture of polyclonal antibodies or a mixture of monoclonal antibodies or a mixture of polyclonal and monoclonal antibodies can be used. In other illustrative embodiments the antibody can be an Fab fragment or an scFv fragment of an antibody (i.e., an Fab fragment or a single chain variable region of an antibody that is directly labeled), or a mixture thereof, capable of preferential binding to cancer cells due to overexpression of the receptors to which these antibodies are directed. Any of the types of antibodies described herein can be used in combination. Any anti-vitamin receptor antibodies known in the art can be used, such as those described in Ross et al., Cancer 73: 2432-2443 (1994), Garin-Chesa et al., Am. J. Pathol. 142(2): 557-67 (1993), Franklin, et al., Int. J. Cancer Suppl. 8: 89-95 (1994) (i.e., monoclonal antibodies 146, 343, 458, and 741), Li et al., J. Nuc. Med. 37:665-672 (1996), Toffoli, Int. J. Cancer 74: 193-198 (1997), Veggian, Tumor 75:510-513 (1989), Weitman, et al., Cancer Research 52: 3396-3401, Bueno et al., J. Thor. Card. Sur. Feb. 121: 225-233 (2001), and Coney, et al., Cancer Research 51: 6125-6132 (1991) (i.e., MOv18 and MOv19).

Any of the antibodies for use in the immunohistochemical methods and kits described herein can be capable of being directly labeled with reagents known to the skilled artisan for direct detection of the antibody (e.g., horse radish peroxidase, alkaline phosphatase, chemiluminescent compounds, and the like) in an immunohistochemical staining method. Alternatively, a second labeled antibody that binds to the anti-vitamin receptor antibody can be used in the immunohistochemical staining method.

In accordance with one embodiment of the invention the antibody can bind with high affinity to receptors on cancer cells or other cell types. The high affinity binding can be inherent to the antibody or the binding affinity can be enhanced by the use of a chemically modified antibody.

Any of these antibody populations for use in the immunohistochemical methods and kits described herein can be purified by standard methods used for purification of proteins. A variety of methods for antibody purification are well-known to those skilled in the art. Typically, a polyclonal antibody is collected from the serum of the animal injected with the antigen, and a monoclonal antibody is collected from the culture medium of the hybridoma cells that secrete the monoclonal antibody or from ascites fluid in animals injected with the hybridoma cells. For purification from serum or culture medium, the antibody can be subjected to a purification or fractionation technique known to those skilled in the art. Conventional purification or fractionation techniques include gel filtration, ion exchange chromatography, DEAE-Sepharose column chromatography, affinity chromatography, solvent-solvent extraction, ultrafiltration, FPLC, and HPLC. Purified antibodies can be concentrated by such techniques as, for example, ultrafiltration and tangential flow filtration. It should be understood that the purification methods described above for purification of antibodies from serum, culture medium, or ascites fluid are nonlimiting and any purification techniques known to those skilled in the art can be used to purify the antibodies if such techniques are required to obtain a purified antibody.

The antibodies used in the immunohistochemical staining assays described herein are used in one embodiment to formulate prognostic compositions comprising effective amounts of the antibody. Examples of compositions that can be used include aqueous solutions of the antibody, for example, in a solution of phosphate-buffered saline, or other buffer solutions known in the art, a saline solution with 5% glucose or other well-known compositions such as alcohols, glycols, esters and amides. The antibodies can be stabilized through the addition of other proteins (e.g., bovine serum albumin, gelatin, and the like) or chemical agents (e.g., glycerol, polyethylene glycol, EDTA, potassium sorbate, sodium benzoate, protease inhibitors, reducing agents, aldehydes, and the like). The antibody compositions in the kits can also be in the form of a reconstitutable lyophilizate comprising the antibody.

The binding site for the antibody can include any receptors preferentially expressed/presented on the surface of or within the cancer cells. The binding site for the antibody may also be present on the surface of activated macrophages or other stimulated immune cells. A surface-presented protein preferentially expressed by these cells is a receptor that is either not present or is present at insignificant concentrations on normal cells providing a means for preferential binding of antibodies to these cells. Accordingly, any receptor that is upregulated on these cells compared to normal cells, or which is not expressed/presented on the surface of normal cells, or any receptor that is not expressed/presented on the surface of normal cells in significant amounts could be used for quantification. In one embodiment the site that binds the antibody is a vitamin receptor, for example, the folate receptor.

In illustrative embodiments of the invention, vitamin receptors constitute the entity that is quantified, either directly (e.g., immunohistochemistry) or indirectly (e.g., using a fluorescence in situ hybridization assay), or detected in accordance with the methods and kits described herein. Acceptable vitamin receptors that can be quantified or detected in accordance with the methods and kits described herein include the receptors for niacin, pantothenic acid, folic acid, riboflavin, thiamine, biotin, vitamin B₁₂, and the lipid soluble vitamins A, D, E and K.

In one illustrative embodiment, the folate receptor can be quantified. Folic acid and its reduced congeners are required for one carbon transfer reactions that are used in the biosynthesis of nucleotide bases, amino acids and other methylated compounds, and consequently, they are needed in larger quantities by proliferating cells. Folates are transported into cells by either a low affinity reduced folate carrier (Km = 10⁻⁵M) or a high affinity folate receptor (Kd = 10⁻¹⁰M). The reduced folate carrier is ubiquitously expressed and constitutes the sole folate uptake pathway for most normal cells. With the exception of kidney and placenta, normal tissues express low or nondetectable levels of the high affinity folate receptor. However, many tumor tissues, including malignant tissues, such as ovarian, breast, uterine, renal, endometrial, bronchial, colon, lung, and brain cancers, and melanomas, lymphomas, and myelomas express significantly elevated levels of the high affinity folate receptor. In fact, it is estimated that 90% of all ovarian carcinomas overexpress this receptor. Also, it has recently been reported that the folate receptor β, the nonepithelial isoform of the folate receptor, is expressed on activated, but not resting synovial macrophages. Thus, Applicants have found that a prognosis can be obtained for a variety of cancers in which vitamin receptor overexpression can be quantified.

The immunohistochemical staining assay described herein can be performed by any immunohistochemical staining assay protocol known in this art such as those described in U.S. Patents Nos. 5,846,739 and 5,989,838. Generally, in one illustrative embodiment, paraffin-embedded tissue sections can be deparaffinized, rehydrated, and blocked. The tissue sections can be fixed prior to the immunohistochemical assay with any fixing agent known in the art, such as formalin, and/or dried in a hot oven before staining. An antigen retrieval step can also be performed. The sections can then be incubated with a primary antibody, washed to remove unbound antibody, and incubated with a secondary antibody, such as an enzyme-linked antibody. The antibody complexes can be incubated with an insoluble chromogen resulting in an insoluble colored precipitate. The sections can then be counterstained for examination using light microscopy.

In another illustrative embodiment, frozen tissue sections can be used and the frozen sections can be fixed in ethanol. The tissue sections can be blocked, for example, with peroxidase in methanol and with Powerblock™ reagent (Biogenics, San Ramon, CA). Antibody staining can then be performed with the DAKO LSAB™-HRP system (DAKO, Carpinteria, CA).

Any reagents known in the art for immunohistochemical staining assays can be used in the method described herein. In an illustrative embodiment, the tissues can be fixed before staining using any fixing agent known in the art, such as ethanol, acetone, or formalin. In other illustrative embodiments, the reagents for performing an immunohistochemical staining assay can include xylene for deparaffinization, graded alcohol wash solutions (e.g., 100 %, 95%, and 70% ethanol solutions) and water for hydration, phosphate-buffered saline or other buffer solutions, peroxidase, CAS Block™ (DAKO, Carpinteria, CA), or Powerblock™ reagent (Biogenics, San Ramon, CA) for blocking, Borg Decloaker Buffer™ (Biocare Medical,Walnut Creek, CA) for antigen retrieval, hematoxylin (Sigma, St. Louis, MO) for counterstaining, a polyclonal antibody directed against a vitamin receptor, a monoclonal antibody directed against a vitamin receptor, and an EnVision⁺™ HRP/DAB⁺ detection kit (DAKO Cytomation, Carpinteria, CA) or a DAKO LSAB™-HRP detection kit (DAKO, Carpinteria, CA), using a biotin-avidin-horseradish peroxidase method with diaminobenzidine as the substrate for antibody staining. Any other reagents known in the art for immunohistochemical staining assays can be used in the method described herein, including any other antibody staining kit known in the art.

The kits for use in the method contain calibration micrographs, described in more detail below, and can also contain any one or more of the reagents described above for use in performing an immunohistochemical staining assay. Any other reagents known in the art for use in performing an immunohistochemical staining assay can also be included in the kits. The kits can also include instructions for using the kit reagents and/or the calibration micrographs to determine a prognosis for a cancer.

The calibration micrographs (i.e., control slides) are prepared from control tissue sections stained using the same immunohistochemical staining assay protocol used to stain the test samples, and from a cancer tissue. Any cancer tissue can be used, and, for example, a slide with a weak, finely granular staining (1+ staining), a coarse, granular staining (2+ staining), and a strong, intense, coarsely granular staining (3+ staining) can be included.

Another method that can be used 1) to quantify vitamin receptor expression (i.e., for purposes of the fluorescence in situ hybridization assay described below "quantifying vitamin receptor expression" means indirect quantification by quantifying vitamin receptor gene amplification) in cancer cells to obtain a prognosis for cancers and to develop an effective treatment regimen for the patient, or 2) to determine the presence of vitamin receptors in cancer cells for selecting patients that should be treated with a therapy that utilizes vitamin receptor targeting, is fluorescence in situ hybridization (FISH). FISH technology can be used to detect gene amplification in cancer cells that overexpress vitamin receptors, such as the receptors for niacin, pantothenic acid, folic acid, riboflavin, thiamine, biotin, vitamin B₁₂, and the lipid soluble vitamins A, D, E and K, where the gene encoding the receptor is amplified. FISH is advantageous because localized amplification can be detected where only a few cells in the specimen are cancerous.

FISH assays are described in detail in U.S. Patents Nos. 6,358,682 and 6,218,529. Typically a FISH assay utilizes formalin fixed, paraffin-embedded cancer tissues, such as those selected from the group consisting of ovarian cancer cells, uterine cancer cells, endometrial cancer cells, breast cancer cells, colorectal cancer cells, brain cancer cells, renal cancer cells, melanoma cells, multiple myeloma cells, lymphoma cells, and lung cancer cells. Formalin-fixed, paraffin-embedded cancer tissues can be treated chemically and/or enzymatically to digest proteins, and can be treated to convert the DNA from double-stranded DNA to single-strand DNA, such as by heating and/or with high salt concentrations. The DNA can then be fixed in the single-stranded form with a fixing agent such as formamide.

The single-stranded, fixed DNA can then be contacted with a hybridization solution, containing a fluorescently-labeled DNA probe. The probe can be complementary to a nucleic acid that encodes the vitamin receptor or the probe can be complementary to a nucleic acid that is complementary to the nucleic acid that encodes the vitamin receptor. The sections can then be incubated under any conditions known in the art that are favorable for hybridization, and washed in a hybridization wash solution. These hybridization solutions and wash solutions for hybridizations are described in Molecular Cloning, 3rd edition, Edited by Sambrook and Russell, 2001, incorporated herein by reference.

The probe can be fluorescently labeled using a fluorescently-tagged ligand (e.g., fluorescein-labeled avidin) which binds to biotin linked to the DNA probe, or the probe can be directly fluorescently labeled, such as with fluorescein or rhodamine. The nuclear DNA can be counterstained, such as with an intercalating fluorescent dye (e.g., 4',6-diamidino-2-phenylidole (DAPI) in Antifade). An epifluorescence microscope can be used for detection of fluorescence. For example, green light will be emitted by a probe labeled with fluorescein and blue light will be emitted by nuclear DNA labeled with DAPI. In this example, nuclei in the tissue section can be scored for the number of green signals on a blue background. This protocol is described in more detail in U.S. Patent No. 6,358,682.

A kit for performing a FISH assay can include a fluorescently-labeled nucleic acid probe, reagents for in situ hybridization, calibration micrographs (i.e., control slides), and instructions for use of the calibration micrographs or the kit, or any combination thereof. The target population for analysis can, for example, be ovarian cancer cells, uterine cancer cells, endometrial cancer cells, breast cancer cells, colorectal cancer cells, brain cancer cells, renal cancer cells, melanoma cells, multiple myeloma cells, lymphoma cells, or lung cancer cells, or any other cancer cells that overexpress vitamin receptors.

The fluorescently-labeled probe in the kit can be labeled directly with any fluorescent compound known in the art to be useful in FISH such as fluorescein, rhodamine, and the like. Alternatively, the probe in the kit can be labeled indirectly, such as where biotin is conjugated to the probe and the fluorescent probe is indirectly fluorescently labeled by incubation with an avidin-fluorescent label conjugate. One of the kit reagents for in situ hybridization can be a counterstain to stain background nuclear DNA (e.g., DAPI).

Calibration micrographs (i.e., control slides) can be made from tissue sections from the tumor or from cell lines. The cells lines can be evenly distributed on the slide. Cell lines may be advantageous because of the uniformity of the cells. A slide with a normal copy number of the vitamin receptor gene, a slide with a high copy number of the vitamin receptor gene, and a slide with a low copy number of the vitamin receptor gene can be used.

In one illustrative embodiment, a cell line derived from the same tissue of the cancer is used. In another illustrative embodiment, the cell line is not a tumor cell line. The control slides can be prepared, for example, by immobilizing the cells in an immobilization material such as agarose, gelatin, pectin, alginate, carrageenan, monomers, polymers, and the like. The immobilization material can be formed by cooling, adding ions, adding a polymerizing agent, adding a cross linking agent, and the like. In another illustrative embodiment the cells can be clotted in plasma, fixed with formalin, and embedded in paraffin. The paraffin can then be sectioned and the section of the paraffin block can be mounted on a slide. Other fixing agents known in the art can be used. Such techniques are described in Diagnostic Molecular Pathology, Vol. 1, IRL Press, N.Y.

A kit similar to the commercially available Oncor INFORM HER-2/*neu* Gene Detection System (Ventana Medical Systems, Gaithersburg, Md., USA; Cat. No: S8000-KIT) or the Abbott Path Vysion™ HER-2/*neu* kit, but for the detection of vitamin receptor gene amplification can be used. The instruction manuals in the Oncor INFORM kit and the Abbott Path Vysion™ kit are included.

In another illustrative embodiment of the invention, a vitamin receptor binding assay is provided for determining a prognosis for a cancer. The method comprises the steps of contacting the cancer cells with a radiolabeled vitamin receptor-binding ligand, or an analog thereof, quantifying the number of vitamin receptors on the cancer cells, and determining a prognosis for the cancer.

In an illustrative embodiment, the vitamin receptor can be a folate receptor. In another illustrative embodiment, the cancer cells can be breast cancer cells. In this embodiment, the breast cancer can comprise node-negative disease, but the invention is not limited to node-negative disease. In yet another illustrative embodiment, the cancer cells can be selected from the group consisting of ovarian cancer cells, uterine cancer cells, endometrial cancer cells, colorectal cancer cells, brain cancer cells, renal cancer cells, melanoma cells, multiple myeloma cells, lymphoma cells, and lung cancer cells, or any other cancer that overexpresses vitamin receptors. In another illustrative embodiment the radiolabeled ligand can be radiolabeled folate, or an analog thereof.

Analogs of folate include folinic acid, pteropolyglutamic acid, and folate receptor-binding pteridines such as tetrahydropterins, dihydrofolates, tetrahydrofolates, and their deaza and dideaza analogs. The terms "deaza" and "dideaza" analogs refers to the art recognized analogs having a carbon atom substituted for one or two nitrogen atoms in the naturally occurring folic acid structure. For example, the deaza analogs include the 1-deaza, 3-deaza, 5-deaza, 8-deaza, and 10-deaza analogs. The dideaza analogs include, for example, 1,5 dideaza, 5,10-dideaza, 8,10-dideaza, and 5,8-dideaza analogs. The foregoing folic acid analogs are conventionally termed "folates," reflecting their capacity to bind to folate receptors. Other folate receptor-binding analogs include aminopterin, amethopterin (methotrexate), N¹⁰-methylfolate, 2-deamino-hydroxyfolate, deaza analogs such as 1-deazamethopterin or 3-deazamethopterin, and 3',5'-dichloro-4-amino-4-deoxy-N¹⁰-methylpteroylglutamic acid (dichloromethotrexate).

Any vitamin receptor binding assay (i.e., a radioreceptor assay) known in the art can be used such as the assay for quantifying soluble vitamin receptors (see Example 11) described in Parker et al., Anal. Biochem. (2005). Any reagents known in the art to be useful for performing a vitamin receptor binding assay can be used, such as a radiolabeled vitamin receptor-binding ligand, or an analog thereof.

Any of these reagents, such as a radiolabeled vitamin receptor-binding ligand, or an analog thereof, can be incorporated into a kit for use in determining a prognosis for a cancer. The kit can also include a calibration table where the calibration table specifies ranges of numbers of vitamin receptors on the cancer cells and the ranges are correlated with a good versus a poor outcome for the cancer. The kit can also include instructions for use of the kit reagents and for use of the calibration table to determine a prognosis for a cancer.

Methods and kits are also provided for determining the presence of vitamin receptors (i.e., detecting vitamin receptors) in cancer cells to select patients that should be treated with a therapy that utilizes vitamin receptor targeting. In illustrative embodiments, the vitamin receptor can be a folate receptor, the cancer cells can be selected from the group consisting of ovarian cancer cells, uterine cancer cells, endometrial cancer cells, colorectal cancer cells, brain cancer cells, renal cancer cells, melanoma cells, multiple myeloma cells, lymphoma cells, and lung cancer cells, or any other cancer that overexpresses vitamin receptors. The cancer tissues for use in the methods can be surgically removed from the patient. In the embodiment where the cancer cells are breast cancer cells, the breast cancer can comprise node-negative disease, but the invention is not limited to node-negative disease. Vitamin receptors can be detected by any of the methods described herein including immunohistochemical staining assays, FISH assays, and vitamin receptor-binding assays employing a radiolabeled ligand.

The therapy that utilizes vitamin receptor targeting can be, for example, a therapy such as that described in U.S. Patent Application Publications Nos. US-2001-0031252-A1; US-2003-0086900-A1; US-2003-0198643-A1; and US-2005-0002942-A1; or PCT International Publication No. WO 03/097647 or a combination of these therapies.

### EXAMPLE 1

### Tissue Samples

A tissue microarray of invasive breast cancers selected from women with divergent clinical outcomes was constructed. Specifically, of the 67 samples included, 34 were obtained from women who were free of recurrence for a minimum of seven years from diagnosis. The other thirty-three specimens came from women whose disease recurred less than 3.5 years after diagnosis. To find markers relevant to node-negative disease, the set was enriched with node-negative samples. All cancers were diagnosed in 1984-1985, assuring sufficient follow-up for the good outcome group.

The patient and tumor characteristics are shown in Table 1. The sample set was constructed to provide roughly equal numbers of women with early versus no (or late) recurrence. As mentioned, node-negative samples predominated to find discriminatory markers in node-negative disease. Fifty-two women had node-negative disease. Fifteen women had node-positive disease. There were 34 T1 and 33 T2 tumors (Tl = 2 cm or less; T2 = 2.1 to 5 cm). Seventy percent of cancers were estrogen receptor positive and 24% of cancers were estrogen receptor negative. Six percent of cancers were unknown with regards to being estrogen receptor positive or negative. Only ten women received adjuvant chemotherapy and three received tamoxifen treatment. Eighty-one percent of cancers were high grade.

Thirty-three women recurred within 3.5 years of diagnosis, the poor outcome group. Their median time to recurrence was 1.9 years. Thirty-four were free of recurrence for a minimum of seven years after diagnosis. Of this good outcome group, 32 had not recurred at a median of 13.7 years of follow-up. Both of the other two recurred at 14 years following their diagnoses. The percent alive patients versus deceased patients for the poor outcome group was 15% versus 85%. For the good outcome group, 76% are alive and 24% were deceased.

**Table 1. Patient and Tumor Characteristics**

| Patient and Tumor Characteristics (n=67) | | | |
|---|---|---|---|
| | | | Percent |
| Tumor Size | | | |
| | T1 | | 34(51%) |
| | T2 | | 33 (49%) |

| Nodal Status | | | |
|---|---|---|---|
| | N- | | 52 (78%) |
| | N+ | | 15 (22%) |

| ER Status | | | |
|---|---|---|---|
| | + | | 47 (70%) |
| | - | | 16 (24%) |
| | Unknown | | 4 (6%) |

| Histology | | | |
|---|---|---|---|
| | Ductal | | 57 (85%) |
| | Lobular | | 6 (9%) |
| | Adenocarcinoma, NOS | | 4 (6%) |

| Grade | | | |
|---|---|---|---|
| 2 | | | 2 (3 %) |
| 3 | | | 11 (16%) |
| 4 | | | 54 (81%) |
| | | | |

| Adjuvant Therapy | | | |
|---|---|---|---|
| | Tamoxifen | | |
| | Yes | | 3 (4%) |
| | | No | 64 (96%) |
| | | | |
| | Chemotherapy | | |
| | | Yes | |
| | | No | |

| Folate Receptor Staining | | | |
|---|---|---|---|
| | 1 | | 11 (17%) |
| | 2 | | 17 (25%) |
| | 3 | | 39 (58%) |

### EXAMPLE 2

### Antiserum Preparation

Polyclonal antiserum (PU-17) to the folate receptor was from Endocyte, Inc. Briefly, bovine milk folate binding protein was purchased from Sigma Chemical Co., and was affinity-purified on an immobilized folic acid column, and emulsified with Freund's adjuvant before being used to vaccinate New Zealand white rabbits according to established procedures. Two weeks after a second boost of the antigen along with incomplete Freund's adjuvant, blood was drawn and the antiserum was collected.

### EXAMPLE 3

### Antibody Purification

### Reagent Preparation

### 1. Collection Buffer: 1 M phosphate, pH 8

To approximately 450 mL of deionized water, 67.2 g of Na₂HPO₄ and 0.367 g of NaH₂PO₄ · H₂O was added. The mixture was stirred until dissolved and was adjusted to pH to 8.0 with 1 N HCl or NaOH. The final volume was brought up to 500 mL with deionized water, and filtered with Nalgene 500 mL 0.22 µm filter unit.

### 2. Wash Buffer: 10 mM phosphate, pH 6.8

Five mL of the 1 M phospate buffer was diluted, pH 8 into approximately 450 mL deionized water. The pH was adjusted to 6.8 with HCl and the final volume was brought up to 500 mL with deionized water. The solution was filtered with a Nalgene 500 mL, 0.22 µm filter unit.

### 3. Elution Buffer: 0.1 M Glycine, pH 2.5

To approximately 450 mL of deionized water, 3.75 g of glycine was added. The solution was stirred until dissolved and the pH was adjusted to 2.5 with HCl. The final volume was brought up to 500 mL with deionized water and was filtered with a Nalgene 500 mL, 0.22 µm filter unit.

### 4. Phosphate Buffered Saline (PBS), pH 7.4

One hundred mL of Gibco10X PBS was added to approximately 850 mL deionized water and was stirred. The pH was adjusted to 7.4 with HCl, and the final volume was brought up to 1L with deionized water. The solution was filtered with a Nalgene 1L, 0.22 µm filter unit.

### 5. 100X BSA/Azide Solution:

One hundred mg/mL of BSA and 10% sodium azide (w/v) was dissolved in PBS, pH 7.4. One gram of BSA and 1 g of sodium azide was added to 10 mL of 1 X PBS, pH 7.4. The solution was mixed well until all the BSA had dissolved. The solution was filtered with a 0.22 µm syringe-driven filter unit.

### FPLC Purification

An FBP-coupled HiTrap^{®} Affinity Column was warmed to room temperature, and the column was washed with 20 mL of Wash Buffer, pH 6.8, at a flow rate of about 5 mL/min to equilibrate. An FPLC system was used for the washing and elution steps of the purification process. A sample of 15 mL of PU-17 was mixed with Wash Buffer, pH 6.8 in a 1:1 ratio. The diluted antiserum was filtered using a 25 mm MCE Syringe Driven Filter Unit, 0.45 µm and a 10 mL tuberculin syringe. The antiserum was injected with a 5 mL tuberculin syringe.

The antiserum was allowed to bind to column for about 10 minutes at room temperature. After this incubation period, another 5 mL of antiserum was loaded onto the column. After loading the antiserum, 10 mL of Wash Buffer, pH 6.8, was added to the column using a 10 mL tuberculin syringe.

After the two 5 mL injections of antiserum had been put on the column, the column was hooked up to the FPLC system. The column was washed with approximately 10 mL of Wash Buffer (Buffer A) at 5 mL/min. The elution was begun after no material was detected in the wash. A flow rate of 5 mL/min was used and the gradient was increased to 100% Elution Buffer (Buffer B). At this point, 1 mL fractions were collected. The A₂₈₀ was monitored, and the fractions that contained eluted antibody were pooled and were saved (usually fractions 5-10).

After the fractions were pooled, the eluted antibody was neutralized by the addition of about 400 µL of Collection Buffer, pH 8. The Collection Buffer was added to the sample slowly and the pH was monitored with pHydrion 6-8 pH paper. The antibody sample was adequately neutralized when the pH reached pH 6.4-7.0.

After all of the antibody had been eluted from the column, the gradient was switched back to Buffer A (0% Buffer B). The column was re-equilibrated with approximately 20 mL of Wash Buffer (Buffer A). After the column was equilibrated, it was disconnected from the FPLC system, and 2 more 5mL injections of antiserum solution were allowed to bind to the column and eluted. The above-described steps were repeated until all antiserum had been run through the column.

### First Antibody Concentration Step

After all of the serum had been purified and the eluted antibody had been neutralized and pooled, the purified antibody was dispensed into Millipore Ultrafree^{®}-4 Biomax 10K NMWL Centrifugal Concentrators. The concentrators were centrifuged at 3200 x g and at 4°C until a final total volume of about 2 mL was reached.

### Buffer Exchange and Second Antibody Concentration Step

The buffer was exchanged using an equilibrated PD-10 column (Bio-Rad Econo-Pac^{®} 10 DG Disposable Chromatography Column) equilibrated with PBS, pH 7.4. About 2 mL of sample was placed in the column reservoir. All of the sample was allowed to enter the column matrix. At this point, 1 mL fractions were collected from the column. PBS was poured to the top of the column reservoir to elute the antibody. The fractions were checked for protein content using a Quartz UV cuvette and an absorbance of 280 on a spectrophotometer (PBS was used to zero the spectrophotometer). All fractions with protein were pooled.

The pooled antibody solution was dispensed into Millipore Ultrafree^{®}-4 Biomax 10K NMWL Centrifugal Concentrators and the antibody was concentrated as described above until a final volume of around 1-2 mL of purified antibody was achieved.

### EXAMPLE 4

### Tissue Microarray Construction

Tissue microarrays were constructed using a custom fabricated device to produce 0.6 mm tissue cores arrayed in a 216 core-capacity recipient block. Multiple cores from each patient tumor block were incorporated into the recipient block in addition to cores of liver as fiducial markers and controls for immunohistochemistry reactions. Immunohistochemistry was performed on tissue microarray sections mounted on charged slides.

### EXAMPLE 5

### Immunohistochemistry

Immunohistochemistry (IHC) was performed by the following assay. IHC was done on formalin-fixed paraffin-embedded sections, cut at 5 microns onto SuperFrost Charged Slides from Fisher. Slides were baked in a 65°C oven for 30-40 minutes prior to staining. Formalin-fixed, paraffin-embedded samples were deparaffinized with 3 changes of xylene and rehydrated in a series of ethanol washes (100%, 95%, then 70% ethanol) to running distilled water. After dewaxing, slides were placed in a BORG Decloaker Buffer in the Biocare Decloaker Unit (Biocare Medical, Walnut Creek, CA). After the sections were cooled, the sections were rinsed well in running distilled water. Visualization was completed on a DAKO Autostainer for this procedure (at room temperature). Sections were incubated with 3% H₂O₂ in ethanol for 5 minutes to inactivate endogenous peroxides. Sections were then incubated in 1:200 PU-17 rabbit primary antibody (from Endocyte, Inc.) for 30 minutes. Sections were rinsed with TBST Wash Buffer. Labelled Polymer Rabbit EnVision+, HRP/DAB+ detection (DAKO Cytomation, Carpinteria, CA) was applied and was allowed to incubate for 15 minutes. The slides were rinsed with TBST Wash Buffer. Sections were then incubated in diaminobenzidine (DAB+) solution (DAKO Cytomation, Carpinteria,CA) for 5 minutes, counterstained with Modified Schmidt's Hematoxylin for 5 minutes, blued in running tap water for 3 minutes, and were mounted and coverslipped.

### EXAMPLE 6

### Digital Imaging

Slides were counterstained with hematoxylin. Digital imaging was performed using a Bliss "Virtual Microscopy" microscope and computer system (Bacus Laboratories, Lombard, IL) consisting of a Zeiss Axioplan microscrope with computer interfaced electronic stage controls and a high resolution 3CCD video camera to produce a virtual slide with core images linked to a Microsoft Access™ database containing relevant tissue core information. Image and data files were stored on shared server space to provide remote access of digital images for scoring the immunohistochemistry results.

### EXAMPLE 7

### Definitions of Grade and Staining Intensity and Statistics

The grading system utilized evaluates the architectural pattern, degree of nuclear atypia, and mitotic rate (Broders, A.C., JAMA 74: 656-664 (1920) and Broders, A.C., Surg. Clin. North America 21: 947-62 (1941)). Grade 1 tumors have predominantly a glandular or papillary growth pattern and slight nuclear pleomorphism. Grade 4 tumors have a predominantly solid growth pattern and marked nuclear pleomorphism. Grade 2 tumors have predominantly glandular or papillary growth with moderate nuclear pleomorphism. Grade 3 tumors have mixed glandular and solid growth with moderate nuclear pleomorphism.

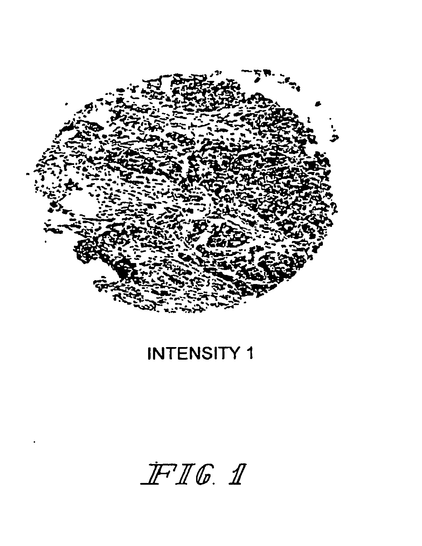

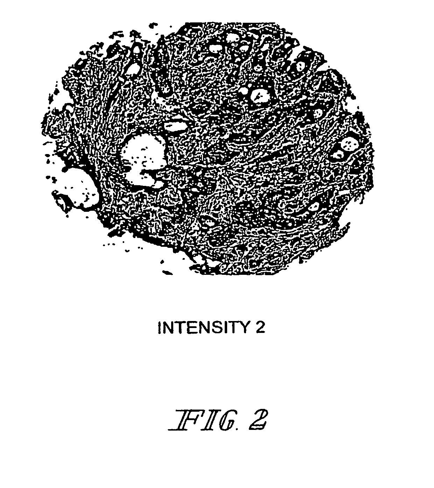

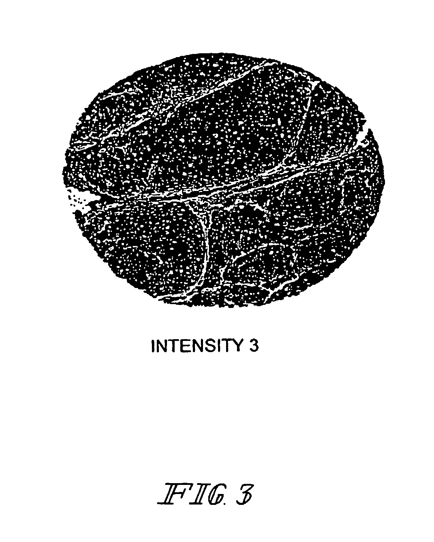

The staining for folate receptor expression was scored 0 to 3. Absence of discernible staining was scored 0. A score of 1 represented weak finely granular staining. A score of 2 reflected a coarser granular staining and 3, a strong intense coarsely granular staining. Positive staining, when present, was typically diffuse throughout the tumor. Representative samples of 1+, 2+, and 3+ staining are shown in Figs. 1-3.

Multiples cores were taken from the large majority of patient samples (4% had six cores, 74% had three cores, 16% had two cores, and 6% had one core). All cores were stained and read. We then averaged the intensity values to arrive at a summary score. No sample had all cores negative. The three summary scores for staining intensity were defined as: 1 = 0.3 - 1.3; 2 = 1.5 - 2.3; 3 = 2.5 - 3.

Descriptive statistics including medians and frequencies were utilized for patient and tumor characteristics. Recurrence-free survival was evaluated using Cox proportional hazards univariate and multivariable modeling.

### EXAMPLE 8

### Receptor Staining Intensity

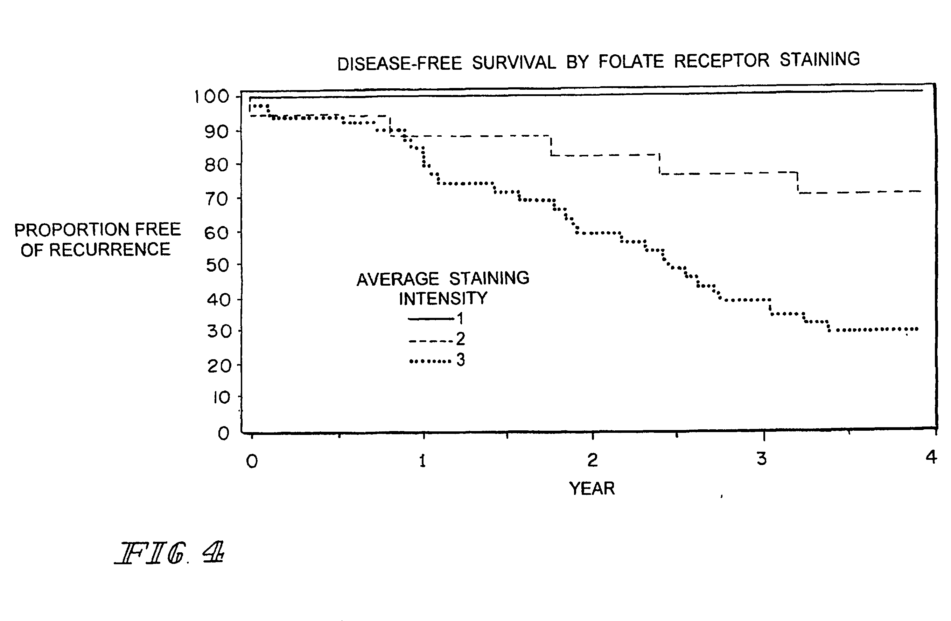

The average folate receptor staining intensity was 1+ in 11 samples (17%), 2+ in 17 samples (25%), and 3+ in 39 (58%) samples. No sample had all cores negative. None of the 11 women with 1+ staining has experienced a recurrence. Of the 17 women with 2+ staining samples, five (29.4%) recurred. In the group with 3+ staining, 28 of the 39 (71.8%) recurred at a median of 2.5 years. The median time to recurrence for the 1+ and 2+ staining groups is greater than four years, as shown in Fig. 4. Figs. 1-3 show examples of 1+, 2+, and 3+ staining. Folate receptor staining in normal tissue controls was negative.

The pattern of folate receptor staining by tumor size, nodal status, grade and estrogen receptor status is shown in Table 2. There was no association between folate receptor overexpression and tumor size, nodal status, or estrogen receptor status. There was an association between higher grade and strong folate receptor expression (p = 0.036).

Comparing various pathologic features versus recurrence as a dichotomous variable (i.e., good versus poor outcome), strong 3+ folate receptor positivity correlated with early recurrence (hazard ratio 6.0; (95% CI 2.3 - 15.7), p<0.001, see Table 3). In this sample set, there were approximately equal numbers of node-positive and node-negative samples in the good and poor outcome groups. Similarly, the proportion of T1 versus T2 tumors and estrogen receptor positive versus estrogen receptor negative samples were relatively evenly distributed between the two outcome groups.

In conclusion, after adjustment for tumor size, nodal status, estrogen receptor status, adjuvant therapy, tumor grade, and histology, 3+ folate receptor staining remained significantly associated with poor outcome, p<0.001. Strong folate receptor positivity was the most significant prognostic factor in this cohort of patients with breast cancer. Accordingly, there is a strong correlation between overexpression of the folate receptor and early recurrence of breast cancer.

**Table 2.**

| | | Folate Receptor | | | |
|---|---|---|---|---|---|
| | | (average intensity) | | | |
| | | 1 (n = 11) | 2 (n = 17) | 3 (n = 39) | p-value* |
| Tumor Size | | | | | |
| | T1 | 8 | 7 | 19 | 0.272 |
| | T2 | 3 | 10 | 20 | |

| Nodal Status | | | | | |
|---|---|---|---|---|---|
| | Negative | 10 | 10 | 32 | 0.087 |
| | Positive | 1 | 7 | 7 | |

| Estrogen Receptor | | | | | |
|---|---|---|---|---|---|
| | ER + | 6 | 13 | 28 | |
| | ER - | 3 | 3 | 10 | 0.711 |
| | Unknown | 2 | 1 | 1 | |

| Grade | | | | | |
|---|---|---|---|---|---|
| | 2 | 2 | 0 | 0 | |
| | 3 | 3 | 3 | 5 | 0.036 |
| | 4 | 6 | 14 | 34 | |

| | | | | | |
|---|---|---|---|---|---|
| *All p values performed via Fisher's Exact Test. | | | | | |

**Table 3. Pathologic Features and Outcome**

| | | Recurrence | | |
|---|---|---|---|---|
| | | Early | Late/Never | p value* |
| Tumor Size | | | | |
| | T1 | 15 | 19 | 0.18 |
| | T2 | 19 | 14 | |

| Nodal Status | | | | |
|---|---|---|---|---|
| | N- | 27 | 25 | 0.82 |
| | N+ | 7 | 8 | |

| Estrogen Receptor | | | | |
|---|---|---|---|---|
| | Not Done | 1 | 3 | 0.51 |
| | + | 26 | 21 | |
| | - | 7 | 9 | |

| Grade | | | | |
|---|---|---|---|---|
| | 2 | 0 | 2 | 0.07** |
| | 3 | 3 | 8 | |
| | 4 | 31 | 23 | |

| Folate Receptor (average intensity) | | | | |
|---|---|---|---|---|
| | 1 | 0 | 11 | < 0.001 |
| | 2 | 5 | 12 | |
| | 3 | 28 | 11 | |

| | | | | |
|---|---|---|---|---|
| *All p-values are chisquare unless otherwise indicated. ** Fisher's Exact test | | | | |

### EXAMPLE 9

### Immunohistochemistry

The immunohistochemical staining assay can be performed by any immunohistochemical staining procedure known in the art. Another illustrative procedure is described below.

### Deparaffinization and Rehydration of Tissue Sections

The slides were placed in 3 successive baths of xylene for 3 minutes in each bath. Excess liquid was tapped off and the slides were placed in 3 successive baths of 100% ethanol for 15 dips in each bath. Excess liquid was tapped off and the slides were placed in 3 successive baths of 95% ethanol for 15 dips in each bath. The excess liquid was tapped off and the slides were placed in deionized water for 3 changes for 30 seconds each.

### Pretreatment with Heat Induced Epitope Retrieval (HIER)

The slides were placed in a staining dish filled with 250 mL of BORG Decloaker reagent. The staining dish was placed in a Decloaking chamber (BioCare Medical, Walnut Creek, CA) for 3 minutes at a pressure of about 17-25 psi and a temperature of about 120°C. The slides were cooled for 10 minutes.

### Peroxidase Blocking

The slides were rinsed briefly in DI water. The excess water was tapped off with an absorbent wipe. The slides were rinsed again in DI water for 1 minute and excess water was tapped off onto an absorbent wipe. Enough ready-to-use Peroxidase Block from the DAKO EnvisonPlus Kit to cover specimen was applied. the slides were incubated for 5 +/- 1 minutes at room temperature. The solutions were drained from the slides. The slides were washed three times in a PBS bath for 3 minutes +/- 30 seconds each.

### CAS Blocking

Excess buffer was tapped off on an absorbent wipe. Enough ready-to-use CAS Block was added to cover the specimens. The slides were incubated for 10 +/- 1 minutes at room temperature. The solution was drained from the slides and excess solution was tapped off on an absorbent wipe.

### Primary Antibody or Negative Control Reagent

Enough diluted (5 µg/mL) primary antibody (PU-17) or negative control reagent (Rabbit IgG) to cover the specimens was added. The specimens were incubated for 30 +/- 1 minutes at room temperature in a humid chamber. The slides were rinsed gently with PBS, flowing in a direction from isotype control to test article. The slides were washed three times in a PBS bath for 3 minutes +/- 30 seconds each wash.

### Peroxidase Labeled Polymer

Excess buffer was tapped off with an absorbent wipe. Enough ready-to-use HRP-Labeled Polymer Secondary Antibody from the DAKO EnvisionPlus Kit was added to cover the specimens. The specimens were incubated for 30 +/- 1 minutes at room temperature in a humid chamber. The solutions were drained from the slides. The slides were washed three times in a PBS bath for 3 minutes +/- 30 seconds for each wash.

### Substrate-Chromogen

Excess buffer was tapped off with an absorbent wipe. Enough of the prepared Liquid DAB substrate-chromogen solution (prepared according to package insert directions) was added to cover the specimens. The specimens were incubated for 5 +/- 1 minutes at room temperature. The solutions were drained from the slides. The slides were rinsed three times with deionized water for 30 seconds each rinse.

### Counterstaining and Dehydration

Excess water was tapped off. The slides were placed in a Harris Hematoxylin bath for one minute. The slides were rinsed with running deionized water for 15 seconds or until the rinse water appeared clear. The slides were quickly dipped once in 0.5% Acid Alcohol and were rinsed with running DI water for 30 seconds. The slides were then placed in 0.2% Ammonia for 40 seconds, and rinsed with running DI water for 15 seconds. The slides were placed in 3 successive baths of 95% ethanol for 15 dips in each bath. The slides were placed in 3 successive baths of 100% ethanol for 15 dips in each bath. The slides were then placed in 3 successive baths of xylene (or equivalent clearing agent) for 1 minute in each bath. The slides were mounted with mounting media.

### INTERPRETATION OF SLIDES

### Positive Control

The positive control tissue should be examined first to ascertain that all reagents are functioning properly. Presence of a brown-colored end-product at the site of the target antigen is indicative of positive reactivity. If the positive control tissue fails to demonstrate positive staining, results with the test specimens should be considered invalid.

### Negative Control

The negative control tissue should be examined after the positive control tissue to verify the specific labeling of the target antigen by the primary antibody. The absence of specific staining in the negative control tissue confirms the lack of antibody cross-reactivity to cells/cellular components. If specific staining occurs in the negative control tissue, results with the test specimen should be considered invalid.

Nonspecific staining, if present, will be of a diffuse appearance. Sporadic light staining of connective tissue may be observed in sections from excessively formalin-fixed tissues. Necrotic or degenerated cells often stain nonspecifically.

### Test Tissue

Test specimens stained with the primary antibody should be examined last. Positive staining intensity should be assessed within the context of any nonspecific background staining of the negative control reagent. The absence of a specific positive staining reaction can be interpreted as no antigen detected.

A morphological review of the tissue should be done on the slide to determine whether an adequate amount of tissue was present and whether the designated tissue was appropriately represented. Samples failing to meet the above standards are rejected from the analysis.

### Staining Intensity

The staining intensity of the test article is judged relative to the intensity of a control slide containing an adjacent section stained with a negative control antibody. Staining of the section labeled with the negative reagent control was considered "background."
"0" indicates no staining relative to isotype background staining.
"1+" indicates weak reactivity, seen as faint or light brown staining. 1+ staining is usually not visible at low magnifications by microscopic examination.
"2+" indicates moderate reactivity, seen as shades of brown staining of intermediate darkness (intensity). 2+ staining may be visible, but not prominent, at low magnifications by microscopic examination.
"3+" indicates strong reactivity, seen as dark brown to black staining. 3+ staining can be easily recognized as obvious positive staining at low magnifications by microscopic examination. Intensity accentuation can be seen in subcellular locations (membrane, cytoplasm and nucleus) at higher magnifications. Interpretation of Staining Intensity

| Score | FR Assessment | Staining Pattern |
|---|---|---|
| 0 | Negative | No staining in tumor cells above background |
| 1+ | Positive | Staining in tumor cells above background |
| 2+ | Positive | Moderate "2+" staining in ≥ 10% of tumor cells |
| 3+ | Positive | Strong "3+" staining in ≥10% of tumor cells |

### EXAMPLE 10

### FISH Assay

Formalin-fixed, paraffin-embedded sections of cancer tissue will be prepared and will be treated chemically and enzymatically to digest proteins. The sections will then be heated at 75°C in the presence of 20 X SSC and formamide to convert DNA from double-stranded DNA to single-strand DNA. The section will then be contacted with a hybridization solution, containing a fluorescently-labeled DNA probe which is complementary to a nucleic acid that encodes the vitamin receptor or to a nucleic acid that is complementary to the nucleic acid that encodes the vitamin receptor. The sections will then be incubated under conditions favorable for hybridization. The sections will be washed in a mixture of 20 X SSC and formamide.

The hybridized probe will be detected using a fluorescently-tagged ligand (e.g., fluorescein-labeled avidin) which binds to biotin linked to the DNA probe. The nuclear DNA will then be counterstained with an intercalating fluorescent dye (e.g., DAPI in Antifade). An epifluorescence microscope will be used for detection of fluorescence and emission of green (fluorescein) and blue light (DAPI) will result. Nuclei in the tissue section will be scored for the number of green signals on a blue background. This protocol is described in more detail in U.S. Patent No. 6,358,682.

### EXAMPLE 11

### Vitamin Receptor-Binding Assay

Tissue specimens from cancer patients (e.g., ovarian cancer patients or breast cancer patients) will be obtained. All sample preparation procedures will be performed at 4°C and will be performed by a published procedure (Parker et al., Anal. Biochem. (2005)).

### Folate Receptor Assay

An exemplary protocol described in this publication was performed as follows for folate receptor quantification. Tissue samples were homogenized in homogenization buffer (10 mM Tris, pH 8.0, 0.02 mg/ml each of leupeptin and aprotinin; 1 ml buffer/50 mg tissue) using a PowerGen 125 homogenizer (Fisher Scientific). Large debris was removed by mild centrifugation (3000 x g for 15 minutes). Membrane pellets were collected by centrifugation at 40,000 x g for 60 minutes and resuspended in solubilization buffer (50 mM Tris, pH 7.4, 150 mM NaCl, 2 mM *n*-octyl-β-D-glucopyranoside, 5 mM EDTA, and 0.02% sodium azide). Insoluble material was removed by a second 40,000 x g 60 minute centrifugation, and the total protein concentration of the supernatants was determined by the bicinchoninic acid (BCA) method (Pierce Chemical). Each sample was then diluted to 0.25 mg/ml in solubilization buffer, and 100 µl was placed inside each of two Microcon-30 microconcentrators (30,000-MW cutoff, Millipore). Samples were then centrifuged at 14,000 x g for 10 minutes at room temperature to pass all of the liquid through the membrane as well as to retain the solubilized receptors on the surface of the microconcentrator membrane. All subsequent centrifugation steps used the same parameters.

Acetate buffer (55 µl of 30 mM acetic acid, pH 3.0, 150 mM NaCl) was added to each microconcentrator, followed by a centrifugation step. Next, 55 µl of phosphate-buffered saline (PBS) was dispensed into each microconentrator, followed by another centrifugation. Then 50 µl of [³H]-folic acid binding reagent (120 nM [³H]-folic acid (Amersham) in 10 mM Na₂PO₄, 1.8 mM KH₂PO₄, pH 7.4, containing 500 mM NaCl, 2.7 mM KC1 and 25 mM *n*-octyl-β-D-glucopyranoside) or 50 µL of a competing reagent (binding reagent plus 120 µM unlabeled folic acid) were added to the appropriate concentrators. Following a 20 minute incubation at room temperature, the concentrators were washed/centrifuged 3 times with 75 µL of 50 mM *n*-octyl-β-D-glucopyranoside, 0.7 M NaCl in PBS, pH 7.4. After the final wash, the retentates containing the solubilized folate receptors were recovered from the membrane surface of the microconcentrators by 2 rinses with 100 µL of PBS containing 4% Triton-X 100^{®}. The samples were then counted in a liquid scintillation counter (Packard Bioscience Co.). CPM values were converted to picomole of folate receptor based upon the CPM of a known standard, and the final results were normalized with respect to the sample protein content.

### Concentration Dependence and Linearity of Assays

The folate receptor assay procedure outlined above was followed with a few modifications. For the concentration dependence assay, a human B-cell lymphoma tissue known to express elevated folate receptor levels was chosen for the analysis, and 50 µg of total membrane-derived protein were assayed per microconcentrator. Five [³H]folic acid binding reagent concentrations (5, 15, 30, 60, and 120 nM) were tested in the presence and absence of a 1000-fold excess of unlabeled folic acid. Using this assay saturation was achieved. To demonstrate saturation of folate receptor binding sites, 25 µg of a metastatic ovarian adenocarcinoma was used as the tissue sample, and [³H]folic acid binding reagent concentrations of 10, 100, 120, 130, and 140 nM were analyzed (+/- 1000-fold excess unlabeled folic acid).

The assay was also shown to exhibit linearity. For the linearity assay 14, 24, 34, and 45 µg of total membrane-derived protein from a human ovarian papillary serous cystadenocarcinoma tissue specimen were analyzed using the 120 nM [³H]folic acid binding reagent solution. Protein concentrations were determined by the BCA Protein Assay (Pierce).

### EXAMPLE 12

### Vitamin Receptor Expression on Cancer Cells

Immunohistochemistry was done on formalin-fixed paraffin-embedded sections of colon, lung, ovary, and endometrial tumors (30-50 tissue sections were examined for each type of tumor tissue), cut at 5 microns onto SuperFrost Charged Slides from Fisher. Slides were baked in a 65°C oven for 30-40 minutes prior to staining. Formalin-fixed, paraffin-embedded samples were deparaffinized with 3 changes of xylene and rehydrated in a series of ethanol washes (100%, 95%, then 70% ethanol) to running distilled water. After dewaxing, slides were placed in a preheated DAKO Target Retrieval Buffer in a 99° C water bath. After the sections were cooled for 20 minutes, the sections were rinsed well in running distilled water. Visualization was completed on a DAKO Autostainer for this procedure (at room temperature). Sections were incubated with 3% H₂O₂ in ethanol for 5 minutes to inactivate endogenous peroxides. Sections were then incubated in a 1:100 dilution of monoclonal antibody 343 primary antibody in DAKO Background Reducing Diluent for 30 minutes. Monoclonal antibody 343 was a gift from Dr. Wilbur Franklin at the University of Colorado and is directed against the folate receptor alpha (Franklin, et al., Int. J. Cancer Suppl., vol. 8: 89-95 (1994)). Sections were rinsed with TBST Wash Buffer. CSAII Biotin-free Tyramide Signal Amplification System (DAKO Cytomation, Carpinteria, CA) was applied and was allowed to incubate for 15 minutes. The slides were rinsed with TBST Wash Buffer. Sections were then incubated in diaminobenzidine (DAB+) solution (DAKO Cytomation, Carpinteria,CA) for 5 minutes, counterstained with Modified Schmidt's Hematoxylin for 5 minutes, blued in running tap water for 3 minutes, and were mounted and coverslipped. The majority of invasive colon, lung, ovary, and endometrial tumors were moderately (2+) to strongly positive (3+) when immunolabeled with the 343 monoclonal antibody against the folate receptor alpha.

### EXAMPLE 13

### Vitamin Receptor Expression on Cancer Cells

Immunohistochemistry was done on formalin-fixed paraffin-embedded sections. Samples were deparaffinized with 3 changes of xylene, rehydrated in a descending ethanol series (99% x 2, 90% x 2), and rinsed in running distilled water. The slides were then placed in DAKO Target Retrieval Buffer (DAKO Cytomation, Cat # S1699) at 99°C for 40 minutes in a standard laboratory water bath, cooled in 50 mM Tris HCl, 300 mM NaCl, 0.1% Tween-20 pH 7.6 (TBST) for 20 minutes at room temperature and then rinsed in TBST for a further for 2 x for 5 minutes.

The sections were then incubated for 5 minutes with the Peroxidase Blocking reagent followed by a 5 minute incubation with a Protein Block reagent, both reagents were included in the CSAll kit (DAKO Cytomation, Cat # K1497). The sections were incubated for 30 minutes with mouse 343 monoclonal antibody (10 µg/ml) in Background Reducing Diluent (DAKO Cytomation, Cat # S3022). The negative control sections were incubated with either non-immune mouse IgG₁ (DAKO Cytomation, Cat # X0931) at 10 µg/ml or Background Reducing Diluent ('no primary' control).

Following incubation with primary antibodies, the sections were then rinsed (3 x 5 minutes) in TBST, incubated with anti-mouse immunoglobulins-HRP for 15 minutes, rinsed in TBST (3 x 5 minutes) and then incubated in the dark with amplification reagent (fluorescyl-tyramide/HRP) for 15 minutes at room temperature. Following the amplification step, the sections were rinsed in TBST (3 x 5 minutes) and then incubated with anti-fluorescein-HRP for 15 minutes at room temperature. After a final series of TBST rinses (3 x 5 minutes), the sections were incubated with diaminobenzidine for 3 minutes. All reagents used during the antibody amplification and visualization steps were supplied as part of the CSA II kit.

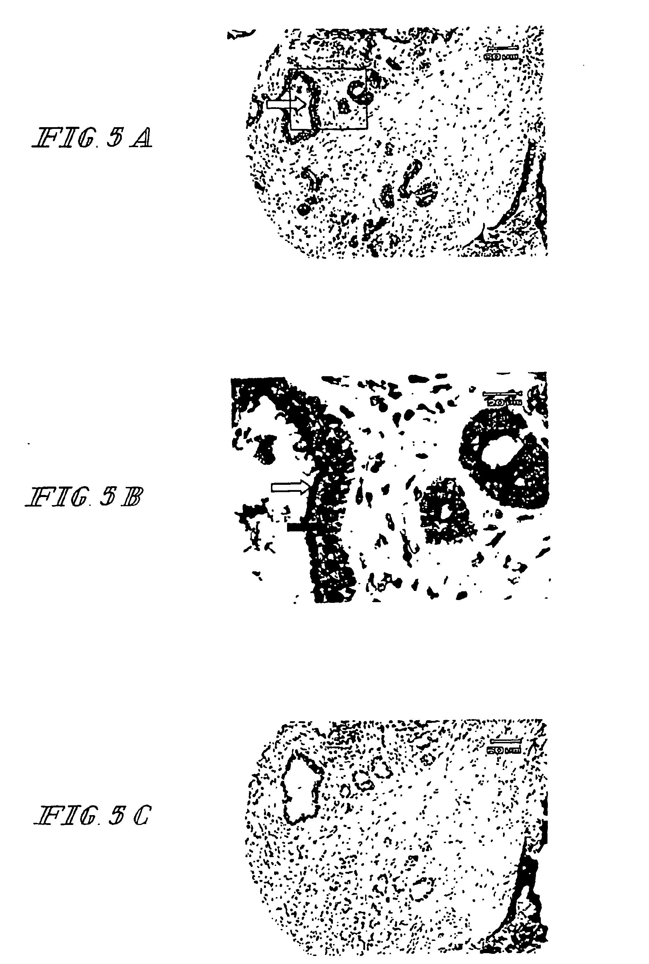

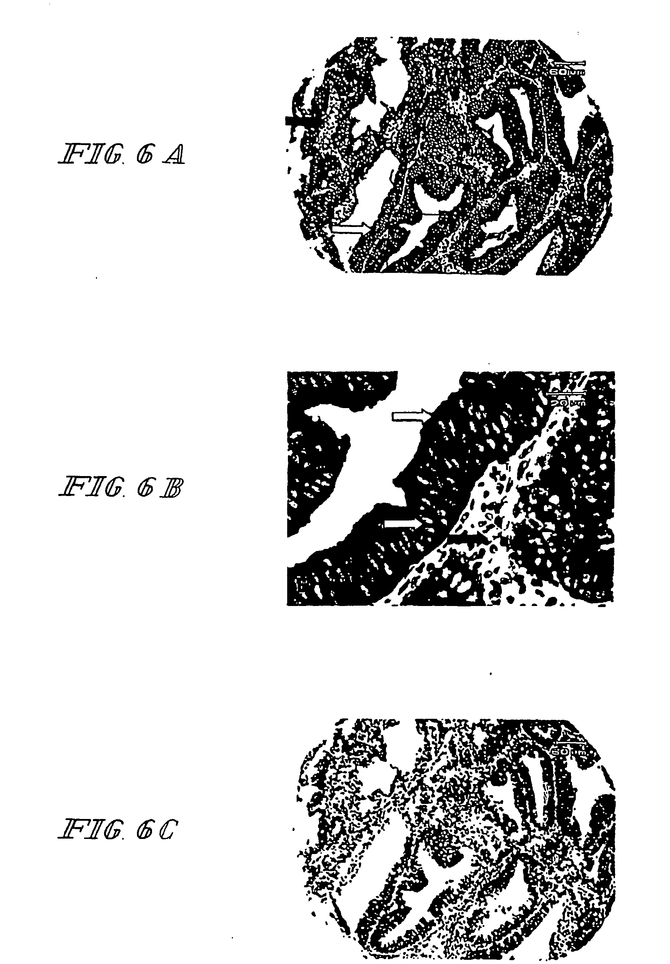

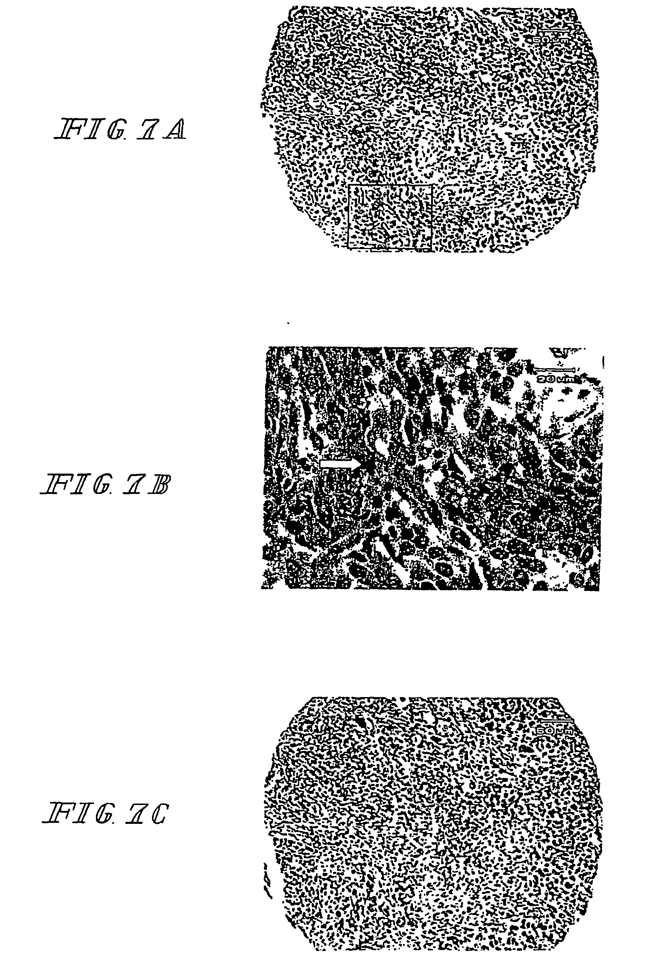

Following chromagenesis, the sections were counterstained with haematoxylin, dehydrated in an ascending series of ethanols (90-99-100%), cleared in two changes of xylene and coverslipped under DePeX. Photomicrographs were acquired using an Olympus BX51 microscope in combination with an Olympus DP12 digital camera. As shown in Figs. 5-7 (panels A, B, and C), pancreatic, endometrial, and cervical cancer tissues stained positively with mAb 343, a monoclonal antibody to the folate receptor alpha.

## Claims

1. A method for determining a prognosis for a cancer by quantifying folate receptor expression on the cancer cells wherein the cancer cells are breast cancer cells, the method comprising the steps of:
quantifying folate receptor expression on the cancer cells in vitro, and
determining a prognosis for the cancer.

2. The method of claim 1 wherein the breast cancer comprises node-negative disease.

3. The method of claim 1 wherein the quantifying step comprises immunohistochemical staining using an antibody.

4. The method of claim 3 wherein the antibody is a polyclonal antibody.

5. The method of claim 3 wherein the antibody is a monoclonal antibody.

6. The method of claim 1 further comprising the step of determining a treatment regimen for the cancer.

7. An immunohistochemical method of determining a prognosis for a cancer, the method comprising the steps of:
contacting the cancer cells in vitro with an antibody directed to a folate receptor,
quantifying folate receptor expression on the cancer cells wherein the cancer cells are breast cancer cells, and
determining a prognosis for the cancer.

8. The immunohistochemical assay of claim 7 wherein the breast cancer comprises node-negative disease.

9. The immunohistochemical assay of claim 7 wherein the antibody is a polyclonal antibody.

10. The immunohistochemical assay of claim 7 wherein the antibody is a monoclonal antibody.

11. A method of determining the presence of folate receptors on cancer cells wherein the cancer cells are breast cancer cells to select patients that should be treated with a therapy that utilizes folate receptor targeting, the method comprising the steps of:
contacting the cancer cells in vitro with an antibody directed to the folate receptor,
quantifying folate receptor expression on the cancer cells, and selecting the patient for treatment with the therapy that utilizes folate receptor targeting.

12. The method of claim 11 wherein the breast cancer comprises node-negative disease.

13. A method for determining a treatment regimen for a cancer patient selected for treatment with a therapy that utilizes folate receptor targeting, the method comprising the steps of:
contacting in vitro cancer cells from the patient with an antibody directed to the folate receptor wherein the cancer cells are breast cancer cells,
quantifying folate receptor expression on the cancer cells, and determining a treatment regimen for the cancer patient.

14. The method of claim 13 wherein the breast cancer comprises node-negative disease.

15. A kit for use in the methods of any preceding claim comprising calibration micrographs wherein the calibration micrographs are derived from breast cancer tissues stained with an antibody to a folate receptor and further comprising an antibody to a folate receptor.

16. The kit of claim 15 further comprising reagents for immunohistochemical staining.

17. The kit of claim 15 wherein the antibody is a polyclonal antibody.

18. The kit of claim 15 wherein the antibody is a monoclonal antibody.

19. The kit of claim 15 wherein the calibration micrographs have staining intensities that correlate with a prognosis for the cancer.

20. The kit of claim 19 wherein the calibration micrographs have staining intensities that correlate with the response of a patient to a folate-targeted therapy.

## Patentansprüche

1. Verfahren zur Bestimmung der Prognose von Krebs durch Quantifizierung der Folatrezeptorexpression auf den Krebszellen, wobei die Krebszellen Brustkrebszellen sind, wobei das Verfahren die Schritte umfasst:
Quantifizierung der Folatrezeptorexpression auf den Krebszellen in vitro, und Bestimmung der Prognose für den Krebs.

2. Verfahren nach Anspruch 1, wobei der Brustkrebs eine knotennegative Erkrankung umfasst.

3. Verfahren nach Anspruch 1, wobei der Quantifizierungsschritt eine immunhistochemische Färbung unter Verwendung eines Antikörpers umfasst.

4. Verfahren nach Anspruch 3, wobei der Antikörper ein polyklonaler Antikörper ist.

5. Verfahren nach Anspruch 3, wobei der Antikörper ein monoklonaler Antikörper ist.

6. Verfahren nach Anspruch 1, weiterhin umfassend den Schritt der Bestimmung einer Behandlungsvorschrift für den Krebs.

7. Immunhistochemisches Verfahren zur Bestimmung der Prognose von Krebs, wobei das Verfahren die Schritte umfasst:
Inkontaktbringen der Krebszellen in vitro mit einem gegen einen Folatrezeptor gerichteten Antikörper,
Quantifizierung der Folatrezeptorexpression auf den Krebszellen, wobei die Krebszellen Brustkrebszellen sind, und
Bestimmung der Prognose auf den Krebs.

8. Immunhistochemischer Assay nach Anspruch 7, wobei der Brustkrebs eine knotennegative Erkrankung umfasst.

9. Immunhistochemischer Assay nach Anspruch 7, wobei der Antikörper ein polyklonaler Antikörper ist.

10. Immunhistochemischer Assay nach Anspruch 7, wobei der Antikörper ein monoklonaler Antikörper ist.

11. Verfahren zur Bestimmung des Vorliegens von Folatrezeptoren auf Krebszellen, wobei die Krebszellen Brustkrebszellen sind, um Patienten zu selektieren, die mit einer Therapie behandelt werden sollen, die das Targeting des Folatrezeptors einsetzt, wobei das Verfahren die Schritte aufweist:
Inkontaktbringen der Krebszellen in vitro mit einem gegen den Folatrezeptor gerichteten Antikörper,
Quantifizieren der Folatrezeptorexpression auf den Krebszellen, und
Auswählen des Patienten zur Behandlung mit der Therapie, die das Targeting des Folatrezeptors einsetzt.

12. Verfahren nach Anspruch 11, wobei der Brustkrebs eine knotennegative Erkrankung umfasst.

13. Verfahren zur Bestimmung einer Behandlungsvorschrift für einen Krebspatienten, ausgewählt aus der Behandlung mit einer Therapie, die das Targeting eines Folatrezeptors einsetzt, wobei das Verfahren die Schritte aufweist:
Inkontaktbringen in vitro von Krebszellen aus dem Patienten mit einem gegen den Folatrezeptor gerichteten Antikörper, wobei die Krebszellen Brustkrebszellen sind, Quantifizieren der Folatrezeptorexpression auf den Krebszellen, und
Bestimmen einer Behandlungsvorschrift für den Krebspatienten.

14. Verfahren nach Anspruch 13, wobei der Brustkrebs eine knotennegative Erkrankung umfasst.

15. Kit zur Verwendung in den Verfahren nach einem der vorhergehenden Ansprüche, umfassend Kalibrierungsmikrobilder, wobei die Kalibrierungsmikrobilder von
Brustkrebsgewebe abgeleitet sind, die mit einem gegen einen Folatrezeptor gerichteten Antikörper gefärbt sind, und weiterhin umfassend einen gegen einen Folatrezeptor gerichteten Antikörper.

16. Kit nach Anspruch 15, weiterhin umfassend Reagentien zur immunhistochemischen Färbung.

17. Kit nach Anspruch 15, wobei der Antikörper ein polyklonaler Antikörper ist.

18. Kit nach Anspruch 15, wobei der Antikörper ein monoklonaler Antikörper ist.

19. Kit nach Anspruch 15, wobei die Kalibrierungsmikrobilder Färbungsintensitäten aufweisen, die mit der Prognose auf Krebs korrelieren.

20. Kit nach Anspruch 19, wobei die Kalibrierungsmikrobilder Färbungsintensitäten aufweisen, die mit der Antwort eines Patienten auf eine Folat-gerichtete Therapie korrelieren.

## Revendications

1. Procédé de détermination d'un pronostic d'un cancer par quantification de l'expression de récepteur de folate sur les cellules cancéreuses, les cellules cancéreuses étant des cellules de cancer du sein, le procédé comprenant les étapes consistant à :
quantifier in vitro l'expression de récepteur de folate sur les cellules cancéreuses, et
déterminer un pronostic du cancer.

2. Procédé selon la revendication 1, dans lequel le cancer du sein comprend une maladie à ganglion négatif.

3. Procédé selon la revendication 1, dans lequel l'étape de quantification comprend une coloration immunohistochimique utilisant un anticorps.

4. Procédé selon la revendication 3, dans lequel l'anticorps est un anticorps polyclonal.

5. Procédé selon la revendication 3, dans lequel l'anticorps est un anticorps monoclonal.

6. Procédé selon la revendication 1, comprenant en outre l'étape de détermination d'un régime de traitement du cancer.

7. Procédé immunohistochimique de détermination d'un pronostic d'un cancer, le procédé comprenant les étapes consistant à :
mettre des cellules cancéreuses en contact in vitro avec un anticorps dirigé vers un récepteur de folate,
quantifier l'expression de récepteur de folate sur les cellules cancéreuses, les cellules cancéreuses étant des cellules de cancer du sein, et
déterminer un pronostic du cancer.

8. Essai immunohistochimique selon la revendication 7, dans lequel le cancer du sein comprend une maladie à ganglion négatif.

9. Essai immunohistochimique selon la revendication 7, dans lequel l'anticorps est un anticorps polyclonal.

10. Essai immunohistochimique selon la revendication 7, dans lequel l'anticorps est un anticorps monoclonal.

11. Procédé de détermination de la présence de récepteur de folate sur des cellules cancéreuses, les cellules cancéreuses étant des cellules de cancer du sein, pour sélectionner des patients qui doivent être traités avec une thérapie qui utilise le ciblage de récepteur de folate, le procédé comprenant les étapes consistant à :
mettre des cellules cancéreuses en contact in vitro avec un anticorps dirigé vers le récepteur de folate,
quantifier l'expression de récepteur de folate sur les cellules cancéreuses, et
sélectionner le patient pour le traitement avec la thérapie qui utilise le ciblage de récepteur de folate.

12. Procédé selon la revendication 11, dans lequel le cancer du sein comprend une maladie à ganglion négatif.

13. Procédé de détermination d'un régime de traitement pour un patient cancéreux sélectionné pour le traitement avec une thérapie qui utilise le ciblage de récepteur de folate, le procédé comprenant les étapes consistant à :
mettre des cellules cancéreuses du patient en contact in vitro avec un anticorps dirigé vers le récepteur de folate, les cellules cancéreuses étant des cellules de cancer du sein,
quantifier l'expression de récepteur de folate sur les cellules cancéreuses, et
déterminer un régime de traitement pour le patient cancéreux.

14. Procédé selon la revendication 13, dans lequel le cancer du sein comprend une maladie à ganglion négatif.

15. Kit d'utilisation destiné à être utilisé dans les procédés selon l'une quelconque des revendications précédentes, comprenant des micrographes d'étalonnage, les micrographes d'étalonnage étant obtenus à partir de tissus cancéreux du sein colorés avec un anticorps dirigé vers un récepteur de folate et comprenant en outre un anticorps dirigé vers un récepteur de folate.

16. Kit selon la revendication 15, comprenant en outre des réactifs pour la coloration immunohistochimique.

17. Kit selon la revendication 15, dans lequel l'anticorps est un anticorps polyclonal.

18. Kit selon la revendication 15, dans lequel l'anticorps est un anticorps monoclonal.

19. Kit selon la revendication 15, dans lequel les micrographes d'étalonnage présentent des intensités de coloration en corrélation avec un pronostic du cancer.

20. Kit selon la revendication 19, dans lequel les micrographes d'étalonnage présentent des intensités de coloration en corrélation avec la réponse d'un patient à une thérapie ciblée sur le folate.
